# EUROPEAN PATENT APPLICATION

(11) **EP 4 606 371 A1**
(43) Date of publication of application: **27.08.2025**
(21) Application number: 23879017.4
(22) Date of filing: 12.10.2023
(51) Int. Cl.: A61K 9/16, A61K 31/437, A61K 47/34, A61P 25/18

(54) **LUMATEPERONE PHARMACEUTICAL COMPOSITION, AND LONG-ACTING MICROSPHERE SUSTAINED-RELEASE FORMULATION AND PREPARATION METHOD THEREFOR**

(30) Priority: 19.10.2022 CN 202211278109
(71) Applicant: Sichuan Kelun Pharmaceutical Research Institute Co., Ltd., Chengdu, Sichuan 611138 (CN)
(72) Inventor: SU, Zhengxing, Chengdu, Sichuan 611138 (CN); DING, Duohao, Chengdu, Sichuan 611138 (CN); BAO, Fei, Chengdu, Sichuan 611138 (CN); ZHOU, Miaomiao, Chengdu, Sichuan 611138 (CN); ZHANG, Xiaohang, Chengdu, Sichuan 611138 (CN); ZHAO, Dong, Chengdu, Sichuan 611138 (CN); LI, Ming, Chengdu, Sichuan 611138 (CN); ZHANG, Chuan, Chengdu, Sichuan 611138 (CN)
(74) Representative: Hoffmann Eitle
(86) International application number: PCT/CN2023/124255
(87) International publication number: WO 2024/083026

(57) **Abstract**

The present invention relates to the field of pharmaceutical formulations. Disclosed are a composition containing Lumateperone active ingredients, and a long-acting sustained-release formulation and a preparation method therefor. A composition containing Lumateperone active ingredients capable of achieving long-acting sustained release is obtained by means of a specific definition on medicinal polymer auxiliary materials; and preparation process conditions are further controlled to obtain a long-acting sustained-release formulation containing Lumateperone active ingredients, so that the sustained release of Lumateperone is implemented **in** one week to two months, and compared with general formulations (for example: capsules), the compliance of patients can be effectively improved; and clinical requirements of stable drug release are satisfied while the drug safety and effectiveness are ensured.

## Description

### Technical Field

The invention belongs to the field of pharmaceutical formulations, and relates to an antipsychotic sustained-release pharmaceutical formulation and a preparation method thereof, in particular to a Lumateperone pharmaceutical composition, and a long-acting microsphere sustained-release formulation and a preparation method thereof.

### Background Art

Mental illness generally refers to psychological disorders, specifically to various abnormal psychological processes, abnormal personality characteristics, and abnormal behavior patterns caused by physical, psychological or social reasons. It manifests as an inability of a person to act in a socially acceptable, appropriate manner, so that the consequences of his/her behaviors are not suitable for both himself/herself and society. Therefore, antipsychotics are often used to treat or alleviate the symptoms.

Among them, the representative drugs of the first-generation antipsychotics involved are chlorpromazine, perphenazine, haloperidol, penfluridol, etc., which can effectively alleviate positive symptoms by blocking dopamine receptors but are almost ineffective for negative symptoms and cognitive impairment. The representative drugs of second-generation antipsychotics are risperidone, clozapine, olanzapine, aripiprazole, quetiapine, ziprasidone, etc., which can block dopamine receptors and 5-hydroxytryptamine receptors, and have a lower incidence of extrapyramidal adverse reactions than most of the first-generation drugs. However, most of the second-generation drugs lead to weight gain as well as glucose and lipid metabolism disorders (obesity). In the past two years, Lumateperone (CAS No.: 313368-91-1, also known as ITI-722), a new antipsychotic drug with a multi-target effect, has been disclosed, which was approved for marketing in the United States in December 2019 for the treatment of adult schizophrenia. Unlike first-generation and second-generation antipsychotics, it generally targets only two neurotransmitters, i.e., 5-hydroxytryptamine and dopamine. Lumateperone also acts on the transmission of glutamine neurotransmitter, and thus not only can improve the positive symptoms of schizophrenia patients (delusions, hallucinations, behavioral abnormalities, etc.), but also is effective for the negative symptoms (dull emotion, poverty of speech, decreased volitional activity, etc.) and depressive symptoms. In addition, compared with the second-generation antipsychotics, Lumateperone overcomes the disadvantages of weight gain and glucose and lipid metabolism disorders, and reduces the probability of weight gain. Studies have shown that Lumiperone can reduce the risk of weight gain by 13% compared with risperidone, a currently used antipsychotic drug.

Currently, among the existing medication practices for antipsychotics, one of the primary reasons for disease relapse is patients' inability to adhere to regular medication schedules. Clinical trials have confirmed that the existing long-acting injectable antipsychotics can significantly reduce the hospitalization rate and treatment interruption rate of patients compared with oral antipsychotics, making them an important treatment method to prevent relapse. By receiving injections at regular intervals, patients do not have to worry about forgetting to take their medication daily, thereby reducing their daily burden. Furthermore, they can avoid concerns about others discovering their medication use in daily life and work, effectively protecting their privacy. Additionally, drug abuse can also be prevented. At present, the second-generation antipsychotic long-acting injections have been used as the first-line treatment drug for patients with schizophrenia during both the acute and maintenance phases. The second-generation antipsychotic long-acting injections available on the market in China include: risperidone microspheres for injection once every two weeks, paliperidone palmitate for injection once a month, and paliperidone palmitate for injection once every three months. The development of Lumateperone long-acting sustained-release formulations is still in the nascent stage.

Prior arts CN113473988A and CN114072150A disclose that Lumateperone in a free form or a pharmaceutically acceptable salt form can be administered by any suitable routes, including orally, parenterally, transdermally or transmucosally, for example, in the form of tablets, capsules, subcutaneous injections, long-acting injections, or oral rapidly disintegrating tablets or films for sublingual or buccal administration. In terms of dose and effect, the FDA instruction indicates that the optimal dose for oral administration is 42 mg/day without dose titration, which puts forward stringent design requirements for the Lumateperone long-acting sustained-release injection: it needs to have the characteristics of minimal burst release, and stable and uniform release, which is also one of the greatest challenges for the Lumateperone long-acting sustained-release injection at present. Currently, no patent or paper literature discloses an effective preparation method for a Lumateperone long-acting sustained-release injection.

Therefore, it is urgent to develop a Lumateperone long-acting formulation with minimal burst release and stable release to give full play to the clinical advantages of Lumateperone in the field of antipsychotics (such as overcoming the shortcomings of weight gain and glucose and lipid metabolism disorders, and improving safety; and simultaneously improving the positive and negative symptoms (dull emotion, poverty of speech, decreased volitional activity, etc.) and depressive symptoms of schizophrenic patients.

### Summary of the Invention

Aiming to overcome the defects in the prior art, provided are a Lumateperone pharmaceutical composition, a long-acting microsphere sustained-release formulation and a preparation method thereof. In the technical solution, through the specific limitation of the pharmaceutical polymeric excipient, a long-acting sustained-release Lumateperone pharmaceutical composition is obtained; and by further controlling the preparation process conditions, a long-acting microsphere sustained-release formulation is obtained, so that a sustained release of Lumateperone for one week to two months is achieved, and compared with general formulations (such as capsules), the compliance of patients can be effectively improved, a stable release of the formulation is ensured, and the safety and effectiveness of the drug are satisfied.

In order to achieve the above technical object, the following technical solutions are provided.

In the first aspect, the invention provides a Lumateperone pharmaceutical composition, comprising a Lumateperone active ingredient and a pharmaceutical polymeric excipient in a weight ratio of 1:(1.5-19); wherein, the pharmaceutical polymeric excipient comprises a glycolide-lactide copolymer (PLGA) with a weight-average molecular weight (M_{W}) of 5,000 to 120,000 Daltons; and in the glycolide-lactide copolymer, a molar ratio of lactic acid units to glycolic acid units is 95:5 to 50:50. According to the invention, the sustained-release period is adjusted by effectively controlling the degradation rate of the glycolide-lactide copolymer, so that the Lumateperone pharmaceutical composition of the invention has a desired sustained-release effect.

Further, the Lumateperone active ingredient comprises a Lumateperone free base and an available salt form thereof (such as Lumateperone p-toluenesulfonate).

Preferably, the glycolide-lactide copolymer has a weight-average molecular weight (M_{W}) of 5,000 to 80,000 Daltons, and more preferably, the glycolide-lactide copolymer has a weight-average molecular weight (M_{W}) of 40,000 to 80,000 Daltons, for example, 60,000 to 80,000 Daltons.

Preferably, in the glycolide-lactide copolymer, the molar ratio of lactic acid units to glycolic acid units is 85:15 to 50:50; and more preferably, the molar ratio of lactic acid units to glycolic acid units is 85:15 to 75:25.

Preferably, the weight ratio of the Lumateperone active ingredient to the pharmaceutical polymeric excipient is 1:(1.5-10); for example, 1:(1.5-9), 1:(2-8), 1:(2-6) or 1:(3-5), and more particularly, 1:1.5, 1:2, 1:3, 1:4, 1:5, 1:6, 1:7, 1:8 or 1:9.

In the second aspect, the invention provides a long-acting sustained-release formulation, comprising a Lumateperone active ingredient and a pharmaceutical polymeric excipient, wherein the Lumateperone active ingredient accounts for 5 to 40% of the total weight of the long-acting sustained-release formulation, and the pharmaceutical polymeric excipient accounts for 60 to 95% of the total weight of the long-acting sustained-release formulation; wherein the pharmaceutical polymeric excipient comprises a glycolide-lactide copolymer with a weight-average molecular weight of 5,000 to 120,000 Daltons; and in the glycolide-lactide copolymer, a molar ratio of lactic acid units to glycolic acid units is 95:5 to 50:50.

Further, the Lumateperone active ingredient comprises a Lumateperone free base and an available salt form thereof (such as Lumateperone *p*-toluenesulfonate).

Preferably, the glycolide-lactide copolymer has a weight-average molecular weight (M_{W}) of 5,000 to 80,000 Daltons, and more preferably, the glycolide-lactide copolymer has a weight-average molecular weight (M_{W}) of 40,000 to 80,000 Daltons, for example, 60,000 to 80,000 Daltons.

Preferably, in the glycolide-lactide copolymer, the molar ratio of lactic acid units to glycolic acid units is 85:15 to 50:50; and more preferably, the molar ratio of lactic acid units to glycolic acid units is 85:15 to 75:25.

Preferably, the weight ratio of the Lumateperone active ingredient to the pharmaceutical polymeric excipient is 1:(1.5-19); and more preferably, the weight ratio of the Lumateperone active ingredient to the pharmaceutical polymeric excipient is 1:(1.5-10); for example 1:(1.5-9), 1:(2-8), 1:(2-6) or 1:(3-5), and more particularly 1:1.5, 1:2, 1:3, 1:4, 1:5, 1:6, 1:7, 1:8 or 1:9.

Further, the long-acting sustained-release formulation is a long-acting sustained-release micro-granule formulation. The long-acting sustained-release micro-granule formulation comprises spherical granules with a particle size of 1 to 1,000 µm and irregular-shaped granules. Preferably, the long-acting sustained-release micro-granule formulation is prepared by a microfluidic method or a homogeneous emulsification method, and is spherical granules with a particle size of 20 to 200 µm, namely the long-acting microsphere sustained-release formulation.

For the long-acting microsphere sustained-release formulation, the drug loading of the Lumateperone active ingredient can be 5-40%. More precisely, the drug loading of the Lumateperone active ingredient is 10-30%.

In the third aspect, the invention provides a method for preparing the long-acting microsphere sustained-release formulation, which adopts a homogeneous emulsification technology and specifically comprises the following steps:
A1. preparation of an oil phase: dissolving the Lumateperone active ingredient and the pharmaceutical polymeric excipient in an organic solvent to obtain the oil phase for later use;
B1. preparation of an aqueous phase: dissolving a surfactant in water to obtain the aqueous phase for later use;
C1. emulsification: taking a certain volume of the aqueous phase obtained in step B1 as an external aqueous phase I; starting high-speed shearing, adding the oil phase obtained in step A1 into the external aqueous phase I, and continuously shearing to obtain an incompletely solidified emulsion or suspension;
D1. solvent evaporation: taking a certain volume of the aqueous phase obtained in step B1 as an external aqueous phase II; starting stirring, placing the incompletely solidified emulsion or suspension obtained in step C1 into the external aqueous phase II, and continuously stirring until the solvent evaporation is finished so as to obtain crude microspheres; and
E1. post-treatment: washing the crude microspheres obtained in step D1 with pure water, sieving, collecting and drying the microspheres to obtain the long-acting microsphere sustained-release formulation.

Preferably, the method comprises the following steps:
A1. preparation of an oil phase: dissolving the Lumateperone active ingredient (the active drug) and the pharmaceutical polymeric excipient (the molecular skeleton) in an organic solvent, completely dissolving the Lumateperone active ingredient and the pharmaceutical polymeric excipient by stirring, vortexing, ultrasonic treatment and the like to form a transparent solution, namely the oil phase, and temporarily storing the oil phase at a temperature of 4 to 10°C for later use;
B1. preparation of an aqueous phase: dissolving a surfactant in water to obtain the aqueous phase, and temporarily storing the aqueous phase at a temperature of 4 to 10°C for later use;
C1. emulsification: taking a certain volume of the aqueous phase obtained in step B1 as an external aqueous phase I (W1); starting a homogenizing and emulsifying machine for high-speed shearing (for example, at a speed of 1,000 to 3,000 rpm), slowly adding the oil phase obtained in step A1 into the external aqueous phase I at a constant speed, and continuously shearing to finally obtain an incompletely solidified emulsion or suspension;
D1. solvent evaporation: taking a certain volume of the aqueous phase obtained in step B1 as an external aqueous phase II (W2); starting stirring (for example, at a rotating speed of 50 to 1,000 rpm), placing the incompletely solidified emulsion or suspension obtained in step C1 into the external aqueous phase II, and continuously stirring at temperature T1 until the solvent evaporation is finished so as to obtain crude microspheres; and
E1. post-treatment: washing the crude microspheres obtained in step D1 with pure water, sieving, collecting and drying the microspheres to obtain the long-acting microsphere sustained-release formulation.

According to the design objective, the mass percentage of the Lumateperone active ingredient in the oil phase can be 2 to 15%, and the concentration of the pharmaceutical polymeric excipient in the oil phase can be 5 to 25%. The mass percentage of the surfactant in the aqueous phase is 0.5 to 5%; and preferably, the mass percentage of the surfactant in the aqueous phase is 0.5 to 2%.

Preferably, in step A1, the organic solvent comprises one or a mixture of any two or more of dichloromethane, dimethyl sulfoxide, methanol, ethyl acetate, trichloromethane, diethyl ether, benzyl alcohol, N,N-dimethylformamide and N,N-dimethylacetamide.

Preferably, in step B1, the surfactant comprises one or a mixture of any two or more of Pluronic F-127, sodium dodecyl sulfonate, sodium dodecyl sulfate and polyvinyl alcohol (PVA).

Preferably, in step C1, the volume ratio of the oil phase to the external aqueous phase I is 1:5 to 1:50. More preferably, the volume ratio of the oil phase to the external aqueous phase I is 1:10 to 1:20.

Preferably, in step D1, the volume ratio of the oil phase to the external aqueous phase II is 1:10 to 1:200. More preferably, the volume ratio of the oil phase to the external aqueous phase II is 1:10 to 1:100.

Preferably, step D1 is performed at temperature T1 of 0 to 40°C, wherein the temperature T1 may be a constant temperature or a temperature gradient within this range. More preferably, the temperature T1 is 3 to 30°C.

In the fourth aspect, the invention provides a method for preparing the long-acting microsphere sustained-release formulation, which adopts a microfluidic technology and specifically comprises the following steps:
A2. preparation of an oil phase: dissolving the Lumateperone active ingredient and the pharmaceutical polymeric excipient in an organic solvent to obtain the oil phase for later use;
B2. preparation of an aqueous phase: dissolving a surfactant in water to obtain the aqueous phase for later use;
C2. injecting the oil phase obtained in step A2 and a portion of the aqueous phase obtained in step B2 into a microfluidic device respectively by using a syringe pump while controlling the flow rates of the oil phase and the aqueous phase to finally obtain an oil-in-water emulsion, collecting and temporarily storing the oil-in-water emulsion in a receiver containing a portion of the aqueous phase obtained in step B2 to obtain semi-solidified microspheres;
D2. solvent evaporation: placing the semi-solidified microspheres obtained in step C2 from the receiver into an evaporator, and continuously stirring until the solvent evaporation is finished to obtain completely solidified microspheres; and
E2. post-treatment: washing the completely solidified microspheres obtained in step D2 with pure water, sieving, collecting and drying the microspheres to obtain the long-acting microsphere sustained-release formulation.

Preferably, the method comprises the following steps:
A2. preparation of an oil phase: dissolving the Lumateperone active ingredient (the active drug) and the pharmaceutical polymeric excipient (the molecular skeleton) in an organic solvent, completely dissolving the Lumateperone active ingredient and the pharmaceutical polymeric excipient by stirring, vortexing, or ultrasonic treatment and the like to form a transparent solution, namely the oil phase, and temporarily storing the oil phase at a temperature of 4 to 10°C for later use;
B2. preparation of an aqueous phase: dissolving a surfactant in water to obtain the aqueous phase, and temporarily storing the aqueous phase at 4 to 10°C for later use;
C2. injecting the oil phase obtained in step A2 (i.e. the dispersed phase) and a portion of the aqueous phase obtained in step B2 (i.e. the continuous phase) into a microfluidic device respectively by using a syringe pump while controlling the flow rates of the oil phase and the aqueous phase to finally obtain the oil-in-water emulsion, collecting and temporarily storing the oil-in-water emulsion in a receiver containing a portion of the aqueous phase obtained in step B2 to obtain semi-solidified microspheres;
D2. solvent evaporation: placing the semi-solidified microspheres obtained in step C2 from the receiver into an evaporator, and continuously stirring at temperature T2 until the solvent evaporation is finished to obtain completely solidified microspheres; and
E2. post-treatment: washing the completely solidified microspheres obtained in step D2 with pure water, sieving, collecting and drying the microspheres to obtain the long-acting microsphere sustained-release formulation.

According to the design objective, the mass percentage of the Lumateperone active ingredient in the oil phase is 2 to 15%, and the concentration (mass percentage) of the pharmaceutical polymeric excipient in the oil phase is 5 to 25%. The mass percentage of the surfactant in the aqueous phase is 0.5 to 5%; and preferably, the mass percentage of the surfactant in the aqueous phase is 0.5 to 2%.

Preferably, in step A2, the organic solvent comprises one or a mixture of any two or more of dichloromethane, dimethyl sulfoxide, methanol, ethyl acetate, trichloromethane, diethyl ether, benzyl alcohol, N,N-dimethylformamide and N,N-dimethylacetamide.

Preferably, in step B2, the surfactant comprises one or a mixture of any two or more of Pluronic F-127, sodium dodecyl sulfonate, sodium dodecyl sulfate and polyvinyl alcohol (PVA).

Preferably, in step C2, the temperature in the microfluidic device is controlled to be 4 to 10°C to ensure the effective formation of the oil-in-water emulsion, and then the formation of the semi-solidified microspheres in the receiver.

Preferably, in step C2, the flow rate ratio of the oil phase to the aqueous phase is controlled to be 1:2 to 1:200. More preferably, the flow rate ratio of the oil phase to the aqueous phase is 1:10 to 1:100.

Preferably, step D2 is performed at temperature T2 of 3 to 40°C, wherein the temperature T2 may be a constant temperature or a temperature gradient within this range. More preferably, the temperature T2 is 3 to 35°C.

The terms involved herein include the following.

Lumateperone active ingredient: referring to the Lumateperone free base or the available salt forₘ thereof.

Available salt form: referring to a pharmaceutically acceptable salt of the Lumateperone free base, such as, but not limited to, hydrochloride, sulfate, hydrobromide, hydroiodide, nitrate, bisulfate, phosphate, acid phosphate, citrate, acetate, oxalate, isonicotinate, lactate, salicylate, acid citrate, tartrate, oleate, tannate, pantothenate, bitartrate, ascorbate, succinate, maleate, gentisate, fumarate, gluconate, glucuronate, formate, benzoate, glutamate, methanesulfonate, ethanesulfonate, benzenesulfonate, p-toluenesulfonate, and pamoate.

Drug loading: referring to the amount of drug loaded *per* unit weight or unit volume of microspheres, in which the amount of drug that can be released is considered as the effective drug loading. Except for the case that the drug is irreversibly bound to a matrix, the drug loading can be regarded as the drug content of the microspheres.

Encapsulation efficiency: referring to the percentage of an encapsulated substance (such as a drug) in the total formulation, reflecting the degree to which the drug is encapsulated by carriers.

The invention has the following beneficial technical effects:
1. According to the invention, through the specific limitation of the pharmaceutical polymeric excipient, a Lumateperone pharmaceutical composition is obtained; and by further controlling the preparation process conditions, a long-acting sustained-release formulation, particularly long-acting sustained-release microspheres, is obtained, so that a sustained release of Lumateperone for one week to two months is achieved, and compared with general formulations (such as capsules), the compliance of patients can be effectively improved, a stable release of the formulation is ensured, and the safety and effectiveness of the drug are satisfied.
2. The invention provides a method suitable for preparing the long-acting microsphere sustained-release formulation based on the homogeneous emulsification technology. Compared with general oral formulations, the prepared long-acting microsphere sustained-release formulation achieves a stable sustained-release for 1 week to 2 months, effectively improves the compliance of patients and improves the clinical therapeutic effect.
3. Based on the microfluidic technology, microspheres with uniform size and good morphology are prepared. By controlling the flow rate ratio of the oil phase to the aqueous phase and controlling the conditions in the microfluidic device, The release of microfluidic Lumateperone microspheres closely approximates zero-order kinetics and remains stable, thus meeting the safety and efficacy requirements of the drug. This approach effectively addresses the issue of unstable release observed with homogeneous emulsified microspheres.

### Brief Description of the Drawings

FIG. 1 is a structural schematic diagram of the Lumateperone long-acting sustained-release microspheres of the invention (left: the Lumateperone long-acting sustained-release microspheres prepared by the microfluidic method; right: the Lumateperone long-acting sustained-release microspheres prepared by the homogeneous emulsification method).
FIG. 2 shows the results of the in vitro release test of the Lumateperone long-acting sustained-release microspheres obtained in Examples 2-4.
FIG. 3 shows the results of the in vitro release test of the Lumateperone long-acting sustained-release microspheres obtained in Examples 5-7.
FIG. 4 shows the results of the in vitro release test of the Lumateperone long-acting sustained-release microspheres obtained in Examples 8-10.
FIG. 5 is a scanning electron microscopy (SEM) image of the Lumateperone long-acting sustained-release microspheres obtained in Example 17.
FIG. 6 is a cross-sectional SEM image of the Lumateperone long-acting sustained-release microspheres obtained in Example 17.
FIG. 7 shows the results of the in vivo release of the Lumateperone long-acting sustained-release microspheres obtained in Examples 14, 15, 17 and 18 in mice.
FIG. 8 shows the results of the in vivo release of the Lumateperone long-acting sustained-release microspheres obtained in Examples 16 and 19-21 in mice.

### Examples

The invention will be further illustrated by the following examples. The examples of the invention are only used to illustrate the technical solutions of the invention, and are not used to limit the scope of the invention. Some non-essential improvements and adjustments can be made by those skilled in the art without violating the spirit of the invention, which shall still be included within the scope of protection of the invention.

### Example 1

In this example, long-acting sustained-release microspheres containing Lumateperone were prepared by using a homogeneous emulsification technology. The preparation method comprises the following steps:
1) 0.52 g of Lumateperone and 2.08 g of PLGA (LA:GA = 95:5, molecular weight 78,000, carboxyl terminated) were weighed, and 11.8 g of dichloromethane was added for dissolution to obtain a clear oil phase solution, which was stored at 10 °C for later use;
2) a 0.5 wt% polyvinyl acetate (PVA) aqueous solution was prepared as an aqueous phase and stored at 10°C for later use;
3) 90 mL of the aqueous phase was taken as an external aqueous phase I, a homogenizing and emulsifying machine was started for high-speed shearing, and the rotating speed was controlled to be 2,000 rpm; the oil phase was transferred into a 20 mL syringe, and then injected into the external aqueous phase I at a constant speed (0.3 mL/s) with a syringe pump for about 30 s; the mixture was continuously sheared for 180 s, and a suspension was obtained after the emulsification was ended;
4) 540 mL of the aqueous phase was taken as an external aqueous phase II, and stirring was started; the suspension obtained in step 3) was poured into the external aqueous phase II, the mixture was continuously stirred and evaporated at 4 °C for 2 h, then the temperature was raised to 25 °C, and the mixture was continuously stirred and evaporated for 2 h to obtain microspheres; and
5) the microspheres obtained in step 4) were filtered via a sieve, washed, collected and dried to obtain a finished microsphere product, namely the Lumateperone long-acting sustained-release microspheres.

The theoretical drug loading of the Lumateperone long-acting sustained-release microspheres was 0.52/(0.52+2.08)=20%, and the in vitro release test performed on the obtained Lumateperone long-acting sustained-release microspheres exhibited an actual microsphere drug loading of 16.34% and an encapsulation efficiency of 81.7%.

### Example 2

In this example, long-acting sustained-release microspheres containing Lumateperone were prepared by using a homogeneous emulsification technology. The preparation method comprises the following steps:
1) 0.52 g of Lumateperone and 2.08 g of PLGA (LA:GA = 50:50, molecular weight 61,000, carboxyl terminated) were weighed, and 11.8 g of dichloromethane was added for dissolution to obtain a clear oil phase solution, which was stored at 10 °C for later use;
2) a 0.5 wt% PVA aqueous solution was prepared as an aqueous phase and stored at 10 °C for later use;
3) 90 mL of the aqueous phase was taken as an external aqueous phase I, a homogenizing and emulsifying machine was started for high-speed shearing, and the rotating speed was controlled to be 2,000 rpm; the oil phase was transferred into a 20 mL syringe, and then injected into the external aqueous phase I at a constant speed (0.3 mL/s) with a syringe pump for about 30 s; the mixture was continuously sheared for 180 s, and a suspension was obtained after the emulsification was ended;
4) 540 mL of the aqueous phase was taken as an external aqueous phase II, and stirring was started; the suspension obtained in step 3) was poured into the external aqueous phase II, the mixture was continuously stirred and evaporated at 4°C for 2 h, then the temperature was raised to 25°C, and the mixture was continuously stirred and evaporated for 2 h to obtain microspheres; and
5) the microspheres obtained in the step 4) were filtered via a sieve, washed, collected and dried to obtain a finished microsphere product, namely the Lumateperone long-acting sustained-release microspheres.

The theoretical drug loading of the Lumateperone long-acting sustained-release microspheres was 0.52/(0.52+2.08)=20%, and the in vitro release test performed on the obtained Lumateperone long-acting sustained-release microspheres exhibited an actual drug loading of 16.02% and the encapsulation efficiency of 80.1%.

### Example 3

In this example, long-acting sustained-release microspheres containing Lumateperone were prepared by using a homogeneous emulsification technology. The preparation method comprises the following steps:
1) 0.52 g of Lumateperone and 2.08 g of PLGA (LA:GA = 75:25, molecular weight 74,000, carboxyl terminated) were taken, and 11.8 g of dichloromethane was added for dissolution to obtain a clear oil phase solution, which was stored at 10°C for later use;
2) a 0.5 wt% PVA aqueous solution was prepared as an aqueous phase and stored at 10°C for later use;
3) 90 mL of the aqueous phase was taken as an external aqueous phase I, a homogenizing and emulsifying machine was started, and the rotating speed was controlled to be 2,000 rpm; the oil phase was transferred into a 20 mL syringe, and then injected into the external aqueous phase I at a constant speed (0.3 mL/s) with a syringe pump for about 30 s; the mixture was continuously sheared for 180 s, and a suspension was obtained after the emulsification was ended;
4) 540 mL of the aqueous phase was taken as an external aqueous phase II, and stirring was started; the suspension obtained in step 3) was poured into the external aqueous phase II, the mixture was continuously stirred and evaporated at 10°C for 2 h, then the temperature was raised to 25°C, and the mixture was continuously stirred and evaporated for 2 h to obtain microspheres; and
5) the microspheres obtained in step 4) were filtered via a sieve, washed, collected and dried to obtain a finished microsphere product, namely the Lumateperone long-acting sustained-release microspheres.

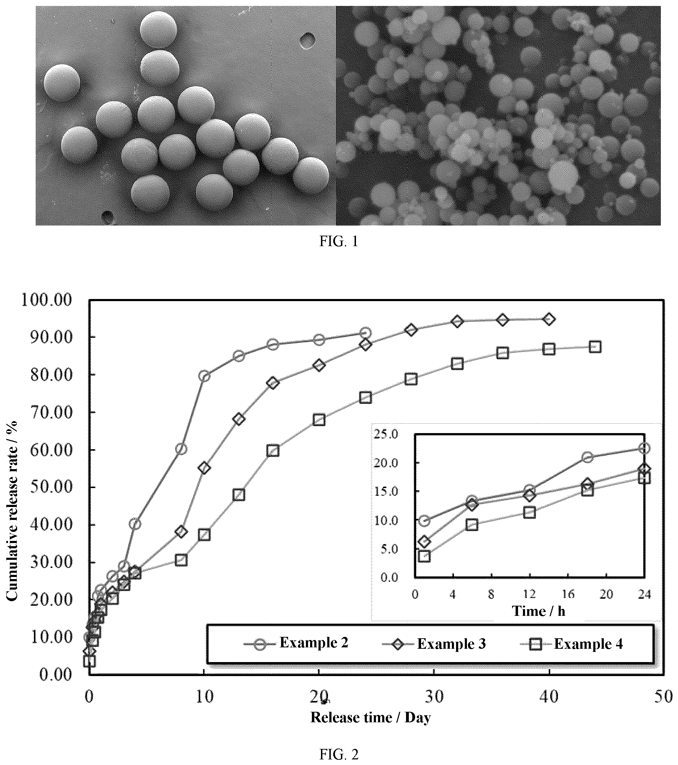

The theoretical drug loading of the Lumateperone long-acting sustained-release microspheres was 0.52/(0.52 + 2.08) = 20%, the actual drug loading was 16.46%, and the encapsulation efficiency was 82.3%. The results of the in vitro release test for the Lumateperone long-acting sustained-release microspheres are shown in FIG. 2.

### Example 4

In this example, long-acting sustained-release microspheres containing Lumateperone were prepared by using a homogeneous emulsification technology. The preparation method comprises the following steps:
1) 0.52 g of Lumateperone and 2.08 g of PLGA (LA:GA = 85:15, molecular weight 72,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase and stored at 10°C for later use;
3) 90 mL of the aqueous phase was taken as an external aqueous phase I, a homogenizing and emulsifying machine was started, and the rotating speed was controlled to be 2,000 rpm; the oil phase was transferred into a 20 mL syringe, and then injected into the external aqueous phase I at a constant speed (0.3 mL/s) with a syringe pump for about 30 s; the mixture was continuously sheared for 180 s, and a suspension was obtained after the emulsification was ended;
4) 540 mL of the aqueous phase was taken as an external aqueous phase II, and stirring was started; the suspension obtained in step 3) was poured into the external aqueous phase II, and the mixture was continuously stirred and evaporated at 10°C for 2 h, then the temperature was raised to 25°C, and the mixture was continuously stirred and evaporated for 2 h to obtain microspheres; and
5) the microspheres obtained in step 4) were filtered via a sieve, washed, collected and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 16.69%, and the encapsulation efficiency was 83.4%. The in vitro release test results of the obtained Lumateperone long-acting sustained-release microspheres are shown in FIG. 2.

### Example 5

In this example, long-acting sustained-release microspheres containing Lumateperone were prepared by using a microfluidic technology. The preparation method comprises the following steps:
1) 0.52 g of Lumateperone and 2.08 g of PLGA (LA:GA = 50:50, molecular weight 61,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase and stored at 10°C for later use;
3) the oil phase (dispersed phase) and the aqueous phase (continuous phase) were injected into a microfluidic device respectively by using syringe pumps. The flow rates were set at 100 µL/h for the oil phase and 2,000 µL/h for the aqueous phase. Within the microfluidic device, the continuous phase sheared the dispersed phase, resulting in an oil-in-water emulsion. Semi-solidified microspheres were collected from this emulsionand subsequently placed in a receiver containing the aqueous phase. Throughout the entire process, the temperature was meticulously maintained at 10°C;
4) after the receiver was full, the semi-solidified microspheres obtained in step 3) were transferred into an evaporator, and stirring was started; a temperature control program (4°C for 1 h, 15°C for 1 h, and 25°C for 2 h) was started, and the organic solvent was removed by evaporation to obtain completely solidified microspheres;
5) the completely solidified microspheres obtained in step 4) were washed with pure water and filtered, and the finished wet microspheres were collected, and dried to obtain a finished microsphere product, namely the Lumateperone long-acting sustained-release microspheres.

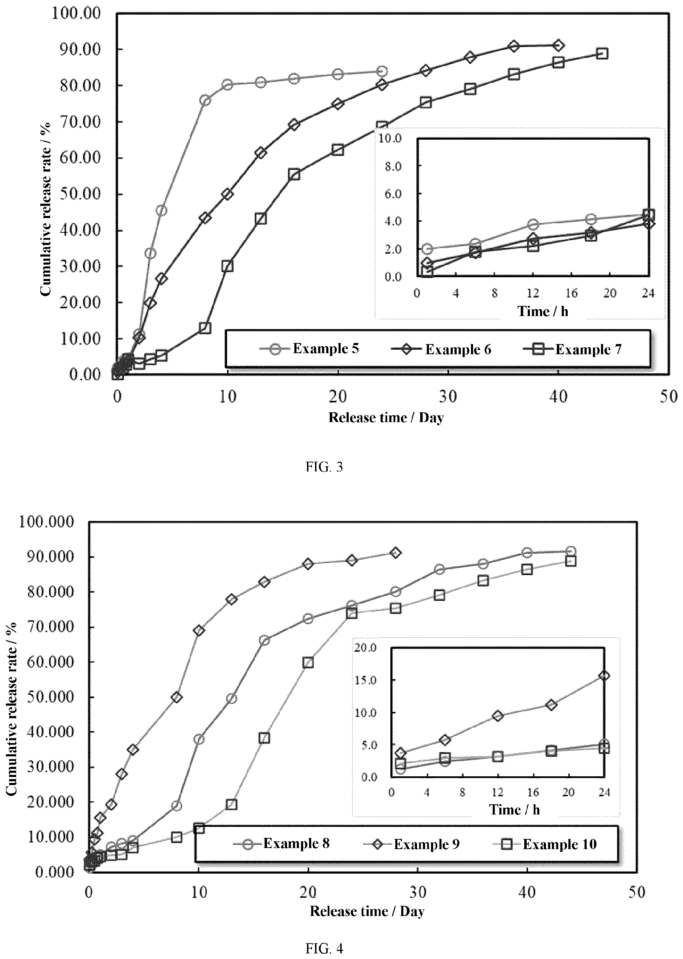

The theoretical drug loading of the Lumateperone long-acting sustained-release microspheres was 0.52/(0.52 + 2.08) = 20%, the actual drug loading was 18.75%, and the encapsulation efficiency was 93.7%. The in vitro release test results of the obtained Lumateperone long-acting sustained-release microspheres are shown in FIG. 3.

### Example 6

In this example, long-acting sustained-release microspheres containing Lumateperone were prepared by using a microfluidic technology. The preparation method comprises the following steps:
1) 0.52 g of Lumateperone and 2.08 g of PLGA (LA:GA = 75:25, molecular weight 74,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) the oil phase (dispersed phase) and the aqueous phase (continuous phase) were injected into a microfluidic device respectively using syringe pumps. The flow rates were set at 100 µL/h for the oil phase and 2,000 µL/h for the aqueous phase, Within the microfluidic device, the continuous phase sheared the dispersed phase, resulting in an oil-in-water emulsion. Semi-solidified microspheres were collected from this emulsion subsequently placed in a receiver containing the aqueous phase. Throughout the entire process, the temperature was meticulously maintained at 10°C;
4) after the receiver was full, the semi-solidified microspheres obtained in step 3) were transferred into an evaporator, and stirring was started; a temperature control program (4°C for 1 h, 15°C for 1 h, and 25°C for 2 h) was started, and the organic solvent was removed by evaporation to obtain completely solidified microspheres; and
5) the completely solidified microspheres obtained in step 4) were washed with pure water and filtered, and the finished wet microspheres were collected and subjected to freeze drying to obtain a finished microsphere product, namely the Lumateperone long-acting sustained-release microspheres.

The theoretical drug loading of the Lumateperone long-acting sustained-release microspheres was 0.52/ (0.52 + 2.08) = 20%, the actual drug loading was 19.30%, and the encapsulation efficiency was 96.5%. The in vitro release test results of the obtained Lumateperone long-acting sustained-release microspheres are shown in FIG. 3.

### Example 7

In this example, long-acting sustained-release microspheres containing Lumateperone were prepared by using a microfluidic technology. The preparation method comprises the following steps:
1) 0.52 g of Lumateperone and 2.08 g of PLGA (LA:GA = 85:15, molecular weight 72,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) the oil phase (dispersed phase) and the aqueous phase (continuous phase) were injected into a microfluidic device respectively using syringe pumps. The flow rates were set at 100 µL/h for the oil phase and 2,000 µL/h for the aqueous phase. Within the microfluidic device, the continuous phase sheared the dispersed phase, resulting in an oil-in-water emulsion. Semi-solidified microspheres were collected from this emulsion subsequently placed in a receiver containing the aqueous phase. Throughout the entire process, the temperature was meticulously maintained at 10°C;
4) after the receiver was full, the semi-solidified microspheres obtained in step 3) were transferred into an evaporator, and stirring was stirred; a temperature control program (4°C for 1 h, 15°C for 1 h, and 25°C for 2 h) was started, and the organic solvent was removed by evaporation to obtain completely solidified microspheres; and
5) the completely solidified microspheres obtained in step 4) were washed with pure water and filtered, and the finished wet microspheres were collected and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 18.98%, and the encapsulation efficiency was 94.9%. The in vitro release test results of the obtained Lumateperone long-acting sustained-release microspheres are shown in FIG. 3.

### Example 8

This example was the same as Example 3, excepting that PLGA terminated with an ester group and having a molecular weight of 74,000 was selected and a homogeneous emulsification technology was used, to prepare Lumateperone long-acting sustained-release microspheres. This technical solution is used for further illustration on the screening of the end group of PLGA.

The theoretical drug loading of the Lumateperone long-acting sustained-release microspheres was 0.52/(0.52 + 2.08) = 20%, the actual drug loading was 18.92%, and the encapsulation efficiency was 94.6%. The in vitro release test results of the obtained Lumateperone long-acting sustained-release microspheres are shown in FIG. 4.

### Example 9

This example was the same as Example 3, excepting that PLGA with a molecular weight of 49,000 was selected and a homogeneous emulsification technology was used, to prepare Lumateperone long-acting sustained-release microspheres. This technical solution is used for further illustration on the screening of the low molecular weight of PLGA.

The theoretical drug loading of the Lumateperone long-acting sustained-release microspheres was 0.52/(0.52 + 2.08) = 20%, the actual drug loading was 19.42%, and the encapsulation efficiency was 97.1%. The in vitro release test results of the obtained Lumateperone long-acting sustained-release microspheres are shown in FIG. 4.

### Example 10

This example was the same as Example 3, excepting that PLGA with a molecular weight of 116,000 was selected, and a homogeneous emulsification technology was used, to prepare Lumateperone long-acting sustained-release microspheres. This technical solution is used for further illustration on the screening of the high molecular weight of PLGA.

The theoretical drug loading of the Lumateperone long-acting sustained-release microspheres was 0.52/(0.52 + 2.08) = 20%, the actual drug loading was 18.04%, and the encapsulation efficiency was 90.2%. The in vitro release test results of the obtained Lumateperone long-acting sustained-release microspheres are shown in FIG. 4.

### Example 11

This example was the same as Example 3, excepting that the feeding amount of Lumateperone was 0.11 g, and a homogeneous emulsification technology was used, to prepare Lumateperone long-acting sustained-release microspheres. This technical solution is used for further illustration on the screening of the low feeding amount of Lumateperone.

The theoretical drug loading of the Lumateperone long-acting sustained-release microspheres was about 0.11/(0.11 + 2.08) = 5%, the actual drug loading was 4.76%, and the encapsulation efficiency was 95.2%.

### Example 12

This example was the same as Example 3, excepting that the feeding amount of Lumateperone was 1.39 g, and a homogeneous emulsification technology was used, to prepare Lumateperone long-acting sustained-release microspheres. This technical solution is used for further illustration on the screening of the high feeding amount of Lumateperone.

The theoretical drug loading of the Lumateperone long-acting sustained-release microspheres was about 1.39/(1.39 + 2.08) = 40%, the actual drug loading was 30.48%, and the encapsulation efficiency was 76.2%.

### Example 13

In this example, long-acting sustained-release microspheres containing a Lumateperone active ingredient was prepared by using a homogeneous emulsification technology. The preparation method comprises the following steps:
1) 0.52 g of Lumateperone and 2.08 g of PLGA (LA:GA = 95:5, molecular weight 42,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) 90 mL of the aqueous phase was taken as an external aqueous phase I, a homogenizing and emulsifying machine was started for high-speed shearing, and the rotating speed was controlled to be 1500 rpm; the oil phase was transferred into a 10 mL syringe, and then injected into the external aqueous phase I at a constant speed (0.3 mL/s) by using a syringe pump for about 30 s; the mixture was continuously sheared for 180 s, and a crude emulsion was obtained after the emulsification was ended;
4) 540 mL of the aqueous phase was taken as an external aqueous phase II, and stirring was started; the crude emulsion obtained in step 3) was poured into the external aqueous phase II, and the mixture was continuously stirred and evaporated at 4°C for 2 h, then the temperature was raised to 25°C, and the mixture was continuously stirred and evaporated for 2 h to obtain microspheres; and
5) the microspheres obtained in step 4) were filtered via a sieve, washed, collected and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 16.61%, the encapsulation efficiency was 83.05%, the particle size was 65.590 µm, and Span was 0.766.

### Example 14

In this example, long-acting sustained-release microspheres containing a Lumateperone active ingredient was prepared by using a homogeneous emulsification technology. The preparation method comprises the following steps:
1) 0.52 g of Lumateperone and 2.08 g of PLGA (LA:GA = 50:50, molecular weight 28000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) 90 mL of the aqueous phase was taken as an external aqueous phase I, a homogenizing and emulsifying machine was started for high-speed shearing, and the rotating speed was controlled to be 2,000 rpm; the oil phase was transferred into a 10 mL syringe, and then injected into the external aqueous phase I at a constant speed (0.3 mL/s) with a syringe pump for about 30 s; the mixture was continuously sheared for 180 s, and a suspension was obtained after the emulsification was ended;
4) 540 mL of the aqueous phase was taken as an external aqueous phase II, and stirring was started; the suspension obtained **in** step 3) was poured into the external aqueous phase II, and the mixture was continuously stirred and evaporated at 4°C for 2 h, then the temperature was raised to 25°C, and the mixture was continuously stirred and evaporated for 2 h to obtain microspheres; and
5) the microspheres obtained in step 4) were filtered via a sieve, washed, collected and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 17.89%, and the encapsulation efficiency was 89.45%.

### Example 15

In this example, long-acting sustained-release microspheres containing a Lumateperone active ingredient was prepared by using a homogeneous emulsification technology. The preparation method comprises the following steps:
1) 0.52 g of Lumateperone and 2.08 g of PLGA (LA:GA = 75:25, molecular weight 74,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) 90 mL of the aqueous phase was taken as an external aqueous phase I, a homogenizing and emulsifying machine was started, and the rotating speed was controlled to be 2,000 rpm; the oil phase was transferred into a 10 mL syringe, and then injected into the external aqueous phase I at a constant speed (0.3 mL/s) with a syringe pump; the mixture was continuously sheared, and a suspension was obtained after the emulsification was ended;
4) 540 mL of the aqueous phase was taken as an external aqueous phase II, and stirring was started; the suspension obtained in step 3) was poured into the external aqueous phase II, the mixture was continuously stirred at a temperature of 10°C, then the temperature was raised to 25°C, and the mixture was continuously stirred to obtain a microsphere; and
5) the microspheres obtained in step 4) were filtered via a sieve, washed, collected and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 17.09%, and the encapsulation efficiency was 85.47%.

### Example 16

In this example, long-acting sustained-release microspheres containing a Lumateperone active ingredient was prepared by using a homogeneous emulsification technology. The preparation method comprises the following steps:
1) 0.52 g of Lumateperone and 2.08 g of PLGA (LA:GA = 85:15, molecular weight 42,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) 90 mL of the aqueous phase was taken as an external aqueous phase I, a homogenizing and emulsifying machine was started, and the rotating speed was controlled to be 2,000 rpm; the oil phase was transferred into a 10mL syringe, and then injected into the external aqueous phase I at a constant speed (0.3mL/s) with a syringe pump for about 30s; the mixture was continuously sheared for 180 s, and a suspension was obtained after the emulsification was ended;
4) 540 mL of the aqueous phase was taken as an external aqueous phase II, and stirring was started; the suspension obtained in step 3) was poured into the external aqueous phase II, and the mixture was continuously stirred and evaporated at 10°C for 2 h, then the temperature was raised to 25°C, and the mixture was continuously stirred and evaporated for 2 h to obtain microspheres; and
5) the microspheres obtained in step 4) were filtered via a sieve, washed, collected and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 17.65%, and the encapsulation efficiency was 88.26%.

### Example 17

In this example, long-acting sustained-release microspheres containing a Lumateperone active ingredient was prepared by using homogeneous emulsification technology. The preparation method comprises the following steps:
1) 0.52 g of Lumateperone and 2.08 g of PLGA (LA: GA = 75:25, molecular weight 51,000, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) the oil phase (dispersed phase) and the aqueous phase (continuous phase) were respectively injected into an emulsifying device using syringe pumps. The flow rates were set at 18 mL/min for the oil phase and 540 mL/min for the aqueous phase, and the oil phase was sheared in an online shearing machine serving as an emulsifying device to obtain a crude emulsion;
4) the crude emulsion obtained in step 3) was transferred into an evaporator, and stirring was started; and a temperature control program (4°C for 1 h, 15°C for 1 h, and 25°C for 2 h) was started, and the organic solvent was removed by evaporation to obtain completely solidified microspheres; and
5) the completely solidified microspheres obtained in step 4) were washed with pure water and filtered, and the finished wet microspheres were collected, and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 17.51%, and the encapsulation efficiency was 87.53%.

### Example 18

In this example, provided is a method for preparing long-acting sustained-release microspheres containing a Lumateperone active ingredient by adopting a homogeneous emulsification technology, which comprises the following steps:
1) 0.52 g of Lumateperone and 2.08 g of PLGA (LA: GA = 75:25, molecular weight 27,500, carboxyl terminated) were weighed, and 11.8 g of dichloromethane was added for dissolution to obtain a clear oil phase solution, which was stored at 10°C for later use;
2) a 0.5 wt% PVA aqueous solution was prepared as an aqueous phase, which was stored at 10°C for later use;
3) the oil phase (dispersed phase) and the aqueous phase (continuous phase) were injected into an emulsifying device respectively using syringe pumps. The flow rates were set at 18 mL/min for the oil phase and 540 mL/min for the aqueous phase, and the oil phase was sheared in an online shearing machine serving as an emulsifying device to obtain a crude emulsion;
4) the crude emulsion obtained in step 3) was transferred into an evaporator, and stirring was started; and a temperature control program (4°C for 1 h, 15°C for 1 h, and 25°C for 2 h) was started, and the organic solvent was removed by evaporation to obtain completely solidified microspheres; and
5) the completely solidified microspheres obtained in step 4) were washed with pure water and filtered, and the finished wet microspheres were collected and dried to obtain a finished microsphere product, namely the Lumateperone long-acting sustained-release microspheres.

The theoretical drug loading of the Lumateperone long-acting sustained-release microspheres was 0.52/(0.52 + 2.08) = 20%, the actual drug loading was 18.18%, and the encapsulation efficiency was 90.89%.

### Example 19

This example was the same as Example 15 except that PLGA (LA:GA = 85:15) terminated with an ester group and having a molecular weight of 41,000 was selected, and 0.26 g of Lumateperone and 2.34 g of PLGA were weighed, to prepare Lumateperone long-acting sustained-release microspheres.

The theoretical drug loading of the Lumateperone long-acting sustained-release microspheres was 0.26 g/(0.26 g + 2.34) = 10%, the actual drug loading was 9.06%, and the encapsulation efficiency was 90.6%.

### Example 20

This example was the same as Example 15 except that PLGA (LA:GA = 85:15) terminated with an ester group and having a molecular weight of 103,000 was selected, 0.13 g of Lumateperone and 2.47 g of PLGA were weighed, and a homogenization and emulsification technology was adopted, to prepare Lumateperone long-acting sustained-release microspheres.

The theoretical drug loading of the Lumateperone long-acting sustained-release microspheres was 0.13/(0.13 + 2.47) = 5%, the actual drug loading was 4.585%, and the encapsulation efficiency was 91.7%.

### Example 21

This example was the same as Example 15 except that PLGA (LA:GA = 85:15) terminated with an ester group and having a molecular weight of 43,000 was selected, 1.04 g of Lumateperone and 1.56 g of PLGA were weighed, and a homogeneous emulsification technology was adopted, to prepare Lumateperone long-acting sustained-release microspheres.

The theoretical drug loading of the Lumateperone long-acting sustained-release microspheres was 1.04/(1.04 + 1.56) = 40%, the actual drug loading was 31.28%, and the encapsulation efficiency was 78.2%.

### Example 22

In the drying procedure of Examples 1-21, freeze drying or vacuum drying can be used, and the freeze drying procedure is shown in Table 1.

**Table 1**

| Stage | Conditions | |
|---|---|---|
| Pre-freezing | Cooling | Room temperature to -30°C × 1 h |
| | Freezing | -30°C×3 h |
| Primary drying | Temperature | -30°C |
| | Pressure | 0.1 mbar |
| | Holding period | 20 h |
| Secondary drying | Pressure | 0.0010 mbar |
| | Heating | -5 to 35°C×150 min |
| | Holding period | 48 h |
| | Cooling | 35 to 25°C×30 min |
| Unboxing | Raising the temperature to 25°C and keeping the temperature until the sample was unboxed. | |

### Experimental example 1

In this experimental example, the Lumateperone long-acting sustained-release microspheres prepared in Examples 1-12 were subjected to in vitro release test [according to the provisions of Chinese pharmacopoeia (P.473)] to further illustrate the invention, which specifically comprises:
1. a 0.01 mol/L physiological isotonic phosphate buffer solution (pH 7.4, containing 0.05% of poloxamer and 0.05% of sodium azide) was prepared;
2. the Lumateperone long-acting sustained-release microspheres prepared in Examples 1-12 were precisely weighed and placed into a test tube, 30 mL of the physiological isotonic phosphate buffer solution was added, and then the test tube was placed in a constant temperature shaker at 37°C for incubation;
3. samples were respectively taken and filtered, and the active drug contents in the sample solution media were respectively measured by a HPLC-UV detection method.

### Conclusion:

1) As demonstrated in the examples of the invention, due to the inherent characteristics of microfluidic technology (where both the drug and excipient form microspheres within the microchannel), there is minimal loss of the active drug, apart from losses due to dissolution and minor losses. Consequently, the microfluidic method offers higher encapsulation efficiency compared to the homogeneous emulsification method when preparing Lumateperone long-acting sustained-release microspheres..
2) According to Figures 2-4, it is evident that Lumateperone long-acting sustained-release microspheres, which release over a period of 1 to 6 weeks, can be prepared using the homogeneous emulsification method and the microfluidic method described in this invention. The microspheres prepared by the microfluidic method exhibit uniform size, favorable morphology (as depicted in Figure 1), minimal burst release, and stable release profiles. By controlling the process conditions, a Lumateperone sustained-release microsphere formulation with reduced burst release and stable release can be achieved.
3) According to FIG. 4, it can be seen that in the preparation process of the Lumateperone long-acting sustained-release microspheres, there is little difference between those using PLGA terminated with an ester group and PLGA terminated with a carboxyl group, and the molecular weight of PLGA has a great impact on the release. By specially limiting the molecular weight of PLGA, the sustained-release period and release curve of the Lumateperone long-acting sustained-release microspheres can be adjusted, and finally, a microsphere formulation meeting clinical requirements can be prepared.
4) In the screening experiments using high (theoretical drug loading: 40%) and low (theoretical drug loading: 5%) feeding amounts of the active ingredient Lumateperone, it is found that although the low feeding amount can ensure a high encapsulation efficiency, the PLGA content in the preparation is too high, which means that the total amount of the solid increases in clinical practice, which will cause pain; and a high feeding amount has adverse effects on the formulation, reduces the encapsulation efficiency, and affects the release behavior to a certain extent.

### Experimental example 2

The Lumateperone microspheres of Examples 14-21 were selected for animal experiments. Several male rats weighing about 250 g were screened and randomly grouped, 5 rats *per* group. The sample was mixed with a solvent (0.5% CMC-Na, 0.1% Tween-20, 0.9% NaCl) evenly. For Examples 14-18 and 21, 10.5 mg/kg of drug was administered intramuscularly; and for Examples 19 and 20, 21 mg/kg of drug was administered intramuscularly, all administered once. Venous blood samples were collected before and after administration to determine the concentration of Lumateperone in plasma after administration.

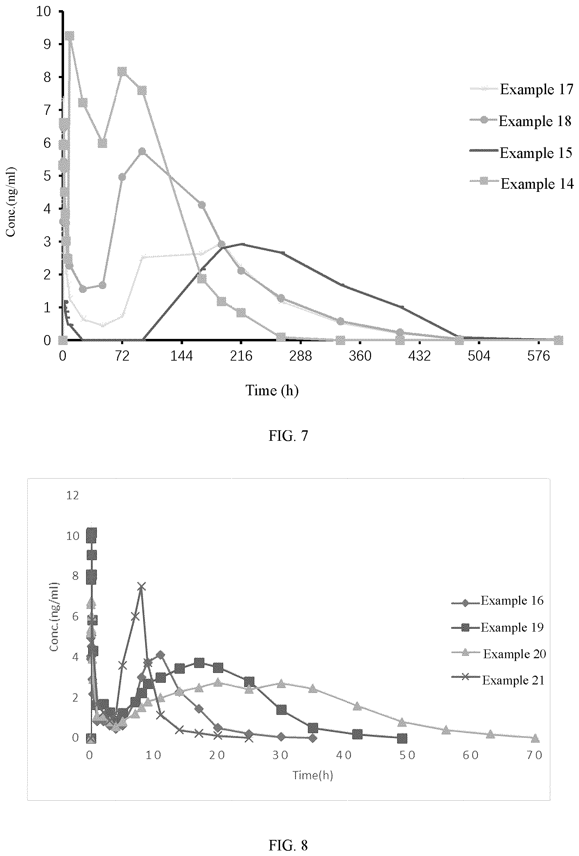

As can be seen from FIGs. 7 and 8, the invention achieved a long-acting sustained-release effect lasting between 12 days to 63 days. It exhibits a minimal burst release, virtually no lag period, and a blood concentration curve that follows a normal distribution.

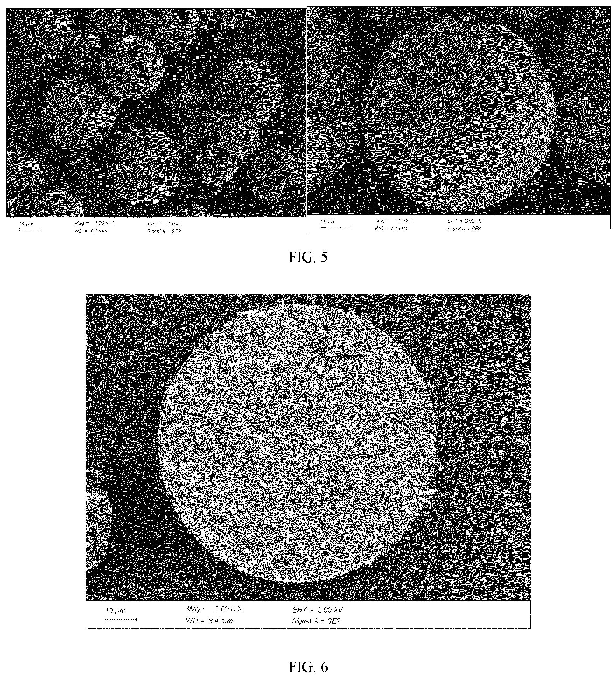

As shown in FIGs. 5 and 6, the microspheres of the invention are spherical **in** shape, uniformly distributed and high compactness inside.

## Claims

1. A Lumateperone pharmaceutical composition, **characterized in that** the Lumateperone pharmaceutical composition comprises a Lumateperone active ingredient and a pharmaceutical polymeric excipient in a weight ratio of 1:(1.5-19); wherein, the pharmaceutical polymeric excipient comprises a glycolide-lactide copolymer with a weight-average molecular weight of 5,000 to 120,000 Daltons; and in the glycolide-lactide copolymer, a molar ratio of lactic acid units to glycolic acid units is 95:5 to 50:50.

2. The Lumateperone pharmaceutical composition according to claim 1, **characterized in that** the Lumateperone active ingredient comprises a Lumateperone free base and an available salt form thereof.

3. The Lumateperone pharmaceutical composition according to claim 1, **characterized in that** the glycolide-lactide copolymer has a weight-average molecular weight of 5,000 to 80,000 Daltons; and in the glycolide-lactide copolymer, the molar ratio of the lactic acid units to the glycolic acid units is 85:15 to 50:50.

4. A long-acting microsphere sustained-release formulation, **characterized in that** the long-acting microsphere sustained-release formulation comprises a Lumateperone active ingredient and a pharmaceutical polymeric excipient, wherein the Lumateperone active ingredient accounts for 5-40% of the total weight of the long-acting microsphere sustained-release formulation, and the pharmaceutical polymeric excipient accounts for 60-95% of the total weight of the long-acting microsphere sustained-release formulation; wherein the pharmaceutical polymeric excipient comprises a glycolide-lactide copolymer with a weight-average molecular weight of 5,000 to 120,000 Daltons; in the glycolide-lactide copolymer, a molar ratio of lactic acid units to glycolic acid units is 95:5 to 50:50;
the long-acting microsphere sustained-release formulation is prepared by a microfluidic method or a homogeneous emulsification method, and the long-acting microsphere sustained-release formulation is spherical particles with a particle size of 20-200 µm.

5. The long-acting microsphere sustained-release formulation according to claim 4, **characterized in that** the Lumateperone active ingredient comprises a Lumateperone free base and an available salt form thereof.

6. The long-acting microsphere sustained-release formulation according to claim 4, **characterized in that** the glycolide-lactide copolymer has a weight-average molecular weight of 5,000 to 80,000 Daltons; and in the glycolide-lactide copolymer, the molar ratio of the lactic acid units to the glycolic acid units is 85:15 to 50:50.

7. A method for preparing the long-acting microsphere sustained-release formulation according to any one of claims 4-6, **characterized in that** the method adopts a homogeneous emulsification method and comprises the following steps:
A1. preparation of an oil phase: dissolving the Lumateperone active ingredient and the pharmaceutical polymeric excipient in an organic solvent to obtain the oil phase for later use;
B1. preparation of an aqueous phase: dissolving a surfactant in water to obtain the aqueous phase for later use;
C1. emulsification: taking a certain volume of the aqueous phase obtained in step B1 as an external aqueous phase I; starting high-speed shearing, adding the oil phase obtained in step A1 into the external aqueous phase I, and continuously shearing to obtain an incompletely solidified emulsion or suspension;
wherein a volume ratio of the oil phase to the external aqueous phase I is 1:5 to 1:50;
D1. solvent evaporation: taking a certain volume of the aqueous phase obtained in step B1 as an external aqueous phase II; starting stirring, placing the incompletely solidified emulsion or suspension obtained in step C1 into the external aqueous phase II, and continuously stirring until the solvent evaporation is finished so as to obtain crude microspheres;
wherein a volume ratio of the oil phase to the external aqueous phase II is 1:10 to 1:200; and
E1. post-treatment: washing the crude microspheres obtained in step D1 with pure water, sieving, collecting and drying the microspheres to obtain the long-acting microsphere sustained-release formulation.

8. The method for preparing the long-acting microsphere sustained-release formulation according to claim 7, **characterized in that** in step A1, the organic solvent comprises one or a mixture of any two or more of dichloromethane, dimethyl sulfoxide, methanol, ethyl acetate, trichloromethane, diethyl ether, benzyl alcohol, N,N-dimethylformamide and N,N-dimethylacetamide.

9. The method for preparing the long-acting microsphere sustained-release formulation according to claim 7, **characterized in that** in step B1, the surfactant comprises one or a mixture of any two or more of Pluronic F-127, sodium dodecyl sulfonate, sodium dodecyl sulfate and polyvinyl alcohol.

10. A method for preparing the long-acting microsphere sustained-release formulation according to any one of claims 4-6, **characterized in that** the method adopts a microfluidic method and comprises the following steps:
A2. preparation of an oil phase: dissolving the Lumateperone active ingredient and the pharmaceutical polymeric excipient in an organic solvent to obtain the oil phase for later use;
B2. preparation of an aqueous phase: dissolving a surfactant in water to obtain the aqueous phase for later use;
C2. injecting the oil phase obtained in step A2 and a portion of the aqueous phase obtained in step B2 into a microfluidic device respectively by using a syringe pump while controlling a flow rate ratio of the oil phase to the aqueous phase to be 1:2-1:200 and controlling a temperature in the microfluidic device to be 4-10°C to obtain an oil-in-water emulsion, collecting the oil-in-water emulsion, and temporarily storing the collected oil-in-water emulsion in a receiver containing a portion of the aqueous phase obtained in step B2 to obtain semi-solidified microspheres;
D2. solvent evaporation: placing the semi-solidified microspheres obtained in step C2 from the receiver into an evaporator, and continuously stirring until the solvent evaporation is finished to obtain completely solidified microspheres; and
E2. post-treatment: washing the completely solidified microspheres obtained **in** step D2 with pure water, sieving, collecting and drying the microspheres to obtain the long-acting microsphere sustained-release formulation.

11. The method for preparing the long-acting sustained-release formulation according to claim 10, **characterized in that** in step A2, the organic solvent comprises one or a mixture of any two or more of dichloromethane, dimethyl sulfoxide, methanol, ethyl acetate, trichloromethane, diethyl ether, benzyl alcohol, N,N-dimethylformamide and N,N-dimethylacetamide.

12. The method for preparing the long-acting sustained-release formulation according to claim 10, **characterized in that** in step B2, the surfactant comprises one or a mixture of any two or more of Pluronic F-127, sodium dodecyl sulfonate, sodium dodecyl sulfate and polyvinyl alcohol.
